# FASCICULE DE BREVET EUROPEEN

(11) **EP 4 142 623 B1**
(45) Date de publication et mention de la délivrance du brevet: **28.01.2026**
(21) Numéro de dépôt: 21721558.1
(22) Date de dépôt: 29.04.2021
(51) Int. Cl.: A61B 17/17, A61B 17/16, A61B 17/68, A61B 17/88, A61B 17/80, A61B 17/86

(54) **ENSEMBLE CHIRURGICAL ADAPTÉ POUR LA RÉDUCTION D'UNE FRACTURE OSSEUSE**
CHIRURGISCHE ANORDNUNG ZUR REDUZIERUNG EINER KNOCHENFRAKTUR
SURGICAL ASSEMBLY DESIGNED TO REDUCE A BONE FRACTURE

(30) Priorité: 29.04.2020 FR 2004250
(43) Date de publication de la demande: 08.03.2023
(73) Titulaire: Newclip International, 2163 Luxembourg (LU)
(72) Inventeur: BALLERINI, Julien, 44100 Nantes (FR); SMITH, Nicolas, NSW, Sydney, Australian Capital Territory 2025 (AU); LARCHE, Grégoire, 49300 Cholet (FR); PODGORSKI, Jean-Pierre, 49230 Sevremoine (FR)
(74) Mandataire: Jacobacci Coralis Harle
(86) Numéro de dépôt international: PCT/EP2021/061292
(87) Numéro de publication internationale: WO 2021/219802

(56) Documents cités:
- US-A1- 2006 264 947
- US-A1- 2009 228 047
- US-A1- 2013 245 699
- US-A1- 2015 127 011
- US-A1- 2017 056 081

## Description

### Domaine technique de l'invention

La présente invention concerne le domaine général du matériel chirurgical, et en particulier le domaine des dispositifs destinés aux techniques d'ostéosynthèse.

Elle concerne plus particulièrement un ensemble chirurgical (ou kit) adapté pour la réduction d'une fracture osseuse, notamment d'une fracture située au niveau des épiphyses osseuses ou articulations.

### Etat de la technique

La consolidation osseuse de certaines fractures nécessite le montage de matériels ou de dispositifs d'ostéosynthèse sur les différents fragments osseux, pour stabiliser et empêcher une mobilité inter fragmentaire excessive.

Les chirurgiens utilisent alors, dans la plupart des cas, des dispositifs d'ostéosynthèse montés par vissage sur les fragments osseux, tels que des vis, broches ou plaques vissées, de sorte à stabiliser ces fragments osseux et par conséquent la fracture.

En particulier, Il existe de nombreux types de plaques associées à un ensemble de vis de fixation, qui sont proposés aux chirurgiens pour réduire une fracture située au niveau des épiphyses osseuses (radius distal, humérus proximal, tibia proximal...).

La plaque support d'ostéosynthèse peut alors comprendre une partie de corps allongée, prolongée par une partie de tête monobloc.

La partie de corps de la plaque support (dite partie diaphysaire de plaque) comprend une pluralité d'orifices traversants adaptés à sa fixation sur le matériau osseux de réception au moyen de vis de fixation ; et la partie de tête de la plaque support (dite partie épiphysaire de plaque) comprend une pluralité d'orifices traversants aptes à autoriser chacun l'accueil d'un organe de fixation allongé, en particulier pour assurer la réduction recherchée de la fracture osseuse.

Très généralement, après réduction anatomique temporaire de la zone traitée au moyen de broches, la ou les broches utilisées sont enlevées pour permettre la pose de vis de verrouillage.

Cependant, la réduction anatomique réalisée peut évoluer lors de l'enlèvement des broches, ou juste après, par manque de stabilité.

Ainsi, d'une manière générale, le matériel et les techniques actuelles à disposition des praticiens/chirurgiens ne permettent pas une stabilisation temporaire efficace ou optimale de la fracture avant fixation avec des vis de verrouillage.

Ils ne permettent pas toujours d'obtenir une grande précision quant à la réduction de la fracture. Et ils nécessitent souvent un temps de mise en œuvre important.

Le document US 2013/245699 A1 est l'état de la technique le plus proche de la présente invention telle que définie dans les revendications.

### Présentation de l'invention

Afin de remédier aux inconvénients précités de l'état de la technique, la présente invention propose un ensemble chirurgical adapté pour la réduction d'une fracture osseuse, en particulier d'une fracture située au niveau des épiphyses osseuses ou articulations, tel que défini dans la revendication 1.

Un tel ensemble chirurgical permet en particulier la mise en place des organes de fixation épiphysaires alors que les broches de fixation, posées pour **la** réduction anatomique de la fracture, restent en place.

D'autres caractéristiques non limitatives et avantageuses de l'ensemble chirurgical conforme à l'invention, prises individuellement ou selon toutes les combinaisons techniquement possibles, sont les suivantes :
- les organes de fixation diaphysaires et les organes de fixation épiphysaires comprennent une même empreinte de tête de sorte à pouvoir être manœuvrés en rotation par le même outil canulé de manœuvre ;
- ledit au moins un guide de visée comprend une empreinte de manœuvre identique à l'empreinte de tête desdits organes de fixation épiphysaires de sorte à pouvoir être manœuvré en rotation par le même outil canulé de manœuvre ;
- l'outil de forage canulé comprend des moyens de mesure pour déterminer la profondeur du forage par rapport à l'extrémité de la broche de fixation sur laquelle le forage est réalisé ;
- l'ensemble chirurgical comprend encore un équipement d'arthroscopie adapté pour permettre une visualisation notamment de la réduction anatomique recherchée et de la mise en place des broches de fixation et des organes de fixation épiphysaires dans le matériau osseux ;
- selon un mode de réalisation, les organes de fixation épiphysaires comprennent (i) une tête d'organe munie d'un filetage externe, lequel filetage externe est adapté pour coopérer avec le taraudage des orifices épiphysaires, et (ii) un corps d'organe, prolongeant ladite tête d'organe, muni (a) d'une partie proximale comportant un filetage externe adapté pour venir se fixer par vissage dans le matériau osseux de réception, et (b) d'une partie distale dépourvue de filetage ;
- selon un autre mode de réalisation, les organes de fixation épiphysaires comprennent (a) une tête d'organe munie d'un filetage externe, lequel filetage externe est adapté pour coopérer avec le taraudage des orifices épiphysaires, et (b) un corps d'organe, prolongeant ladite tête d'organe, lequel corps d'organe est muni d'un filetage sur toute sa longueur ;
- selon encore un autre mode de réalisation, les organes de fixation épiphysaires comprennent (a) une tête d'organe munie d'un filetage externe, lequel filetage externe est adapté pour coopérer avec le taraudage des orifices épiphysaires, et (b) un corps d'organe prolongeant ladite tête d'organe, lequel corps d'organe est lisse, dépourvu de filetage ;
- l'ensemble chirurgical comprend un outillage de type perceuse ou moteur chirurgical adapté pour enfoncer les broches de fixation dans le matériau osseux de réception ;
- l'ensemble chirurgical comprend un premier type de guide de visée d'une longueur L1, et au moins un deuxième type de guide de visée d'une longueur L2 différente de L1.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent des formes, non limitatives, de réalisation de l'invention et où :
[Fig. 1] est une vue en perspective d'une plaque support d'ostéosynthèse faisant partie de l'ensemble chirurgical proposé ;
[Fig. 2] est une vue de côté d'une forme de réalisation possible d'un organe de fixation diaphysaire faisant partie de l'ensemble chirurgical proposé ;
[Fig. 3] est une vue en perspective, selon un premier angle, d'une forme de réalisation possible d'un organe de fixation épiphysaire faisant partie de l'ensemble chirurgical proposé ;
[Fig. 4] montre l'organe de fixation épiphysaire de la figure 3, vu en perspective selon un second angle ;
[Fig. 5] est une vue en perspective, selon un premier angle, d'une première forme de réalisation d'un guide de visée faisant partie de l'ensemble chirurgical proposé ;
[Fig. 6] montre le guide de visée de la figure 5, vu en perspective selon un second angle ;
[Fig. 7] est une vue en perspective, selon un premier angle, d'une deuxième forme de réalisation d'un guide de visée faisant partie de l'ensemble chirurgical proposé ;
[Fig. 8] montre le guide de visée de la figure 7, vu en perspective selon un second angle ;
[Fig. 9] est une vue de côté d'une broche de fixation faisant partie de l'ensemble chirurgical proposé ;
[Fig. 10] est une vue en perspective d'une forme de réalisation possible d'un outil canulé de manoeuvre et de mesure faisant partie de l'ensemble chirurgical proposé ;
[Fig. 11] est une vue en perspective d'une forme de réalisation possible d'un outil de forage canulé faisant partie de l'ensemble chirurgical proposé ;
[Fig. 12] est une vue en perspective illustrant l'opération de positionnement des guides de visée sur la partie épiphysaire de la plaque support fixée sur le matériau osseux de réception ;
[Fig. 13] est une vue en perspective illustrant l'opération de positionnement des broches de fixation sur la partie épiphysaire de la plaque support ;
[Fig. 14] est une vue en perspective illustrant l'opération de mesure de profondeur pour définir la longueur d'un organe de fixation épiphysaire à utiliser pour réduire la fracture osseuse, suivie de l'opération d'enlèvement du guide de visée associé ;
[Fig. 15] est une vue en perspective illustrant l'opération de forage en vue de la mise en place d'un organe de fixation épiphysaire ;
[Fig. 16] est une vue en perspective illustrant l'opération de mise en place d'un organe de fixation épiphysaire ;
[Fig. 17] est une vue en perspective montrant la plaque support équipée de tous les organes de fixation épiphysaires nécessaires à la réduction de la fracture osseuse en présence ;
[Fig. 18] est une vue en perspective illustrant l'opération de forage en vue de la mise en place d'un organe de fixation épiphysaire, cela au moyen d'une variante de réalisation d'un outil de forage canulé comportant des moyens de mesure adaptés pour déterminer la profondeur de forage.

### Ensemble chirurgical

Tel qu'illustré sur les figures annexées, l'ensemble chirurgical selon l'invention qui est proposé au praticien/chirurgien pour la réduction d'une fracture située au niveau des épiphyses osseuses (en particulier une fracture comminutive), comprend notamment une plaque support d'ostéosynthèse, un jeu d'organes de fixation diaphysaires, un jeu d'organes de fixation épiphysaires, un jeu de broches de fixation, un jeu de guides de visée, un outil (ou ancillaire) de manoeuvre en rotation desdits organes de fixation et desdits guides de visée, faisant également office d'ancillaire de mesure (pour déterminer la longueur d'au moins certains des organes de fixation à utiliser), ainsi qu'un matériel de forage (pour le forage d'orifices adaptés au positionnement d'au moins certains des organes de fixation).

De préférence, cet ensemble chirurgical comporte encore un équipement d'arthroscopie (non représenté) adapté pour que le praticien/chirurgien puisse visualiser la réduction anatomique recherchée de la zone articulaire, et aussi l'implantation en cours des broches de fixation, la réalisation des forages et la mise en place des organes de fixation, notamment des organes de fixation épiphysaires.

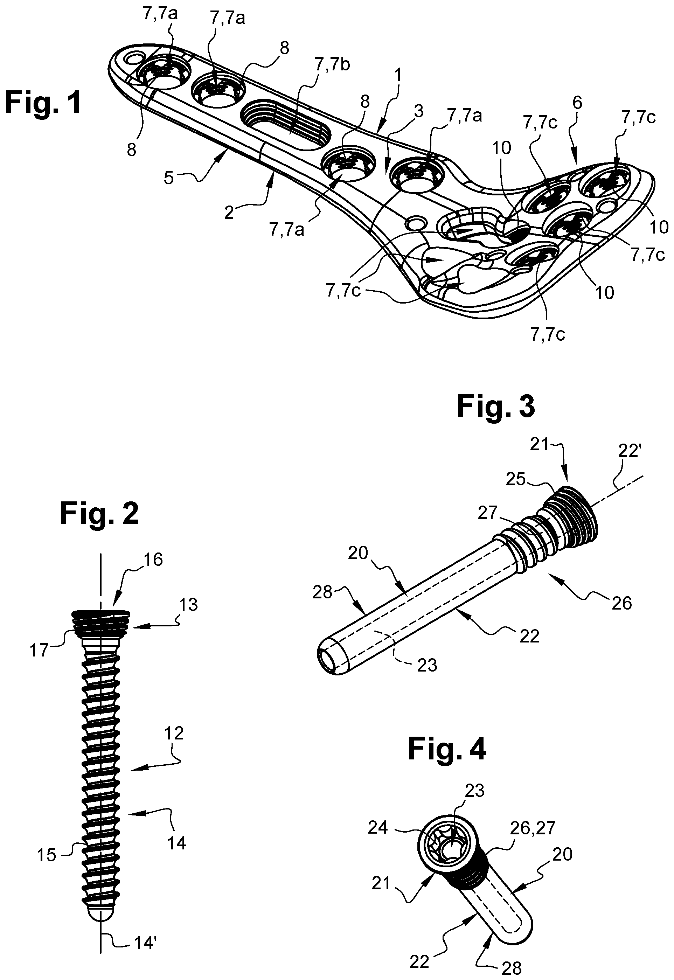

La plaque support d'ostéosynthèse 1 illustrée isolément sur la figure 1, a quelques millimètres d'épaisseur et elle est délimitée par une face inférieure 2 destinée à venir en contact avec le matériau osseux de réception et par une face supérieure 3 opposée.

Cette plaque support 1 comprend une partie de corps allongée 5, dite partie diaphysaire de plaque 5, adaptée pour venir se positionner sur la diaphyse du matériau osseux de réception, et une partie de tête 6, monobloc, dite partie épiphysaire de plaque 6, adaptée pour venir se positionner sur l'épiphyse osseuse, au niveau de la fracture à réduire.

La plaque support 1 est munie d'une pluralité d'orifices 7 qui traversent son épaisseur, entre sa face inférieure 2 et sa face supérieure 3, et qui sont destinés au passage d'organes de fixation, pour sa fixation sur le matériau osseux de réception et aussi, pour certaines, pour la réduction recherchée de la fracture osseuse.

La partie diaphysaire de plaque 5 comprend une pluralité d'orifices diaphysaires 7a, 7b destinés au passage d'organes de fixation diaphysaires pour la fixation de la plaque support 1 sur le matériau osseux de réception.

Ici, quatre de ces orifices diaphysaires 7a ont une forme générale cylindrique. De préférence ces orifices diaphysaires 7a comportent un taraudage interne 8 adapté pour coopérer avec un filetage complémentaire ménagé sur la tête des organes de fixation destinés à leur être associés.

Ici, encore, l'un de ces orifices diaphysaires 7b présente une forme allongée oblongue. Cet orifice diaphysaire 7b oblong est utilisé pour régler le positionnement de la plaque support 1 sur la diaphyse de l'os avant le positionnement des organes de fixation dans les orifices diaphysaires cylindriques 7a.

La partie épiphysaire de plaque 6 comprend quant à elle une pluralité d'orifices épiphysaires 7c arrangés ici sur deux lignes sensiblement perpendiculaires à l'axe de la partie diaphysaire de plaque 5. Dans l'exemple de réalisation représenté, la ligne distale (d'extrémité) est composée d'un alignement de quatre orifices 7c alors que la ligne proximale est composée d'un alignement de trois orifices 7c.

Ici, ces orifices épiphysaires 7c ont une forme générale cylindrique. De préférence ces orifices épiphysaires 7c comportent un taraudage interne 10 adapté pour coopérer avec un filetage complémentaire ménagé sur la tête des organes de fixation destinés à leur être associés.

Une forme de réalisation possible d'organe de fixation diaphysaire 12 est illustrée sur la figure 2.

Cet organe de fixation diaphysaire 12 se présente sous la forme d'une vis de fixation comprenant - une tête de vis 13 dont une partie au moins du contour est destinée à venir en contact avec une partie du pourtour de l'un des orifices diaphysaire 7a, 7b, et - un corps de vis 14, d'axe longitudinal 14', muni d'un filetage 15 pour son ancrage dans le matériau osseux de réception.

Une empreinte de tête 16 (ou empreinte de manœuvre 16), en creux, réalisée dans la tête de vis 13, permet les manœuvres de rotation de la vis de fixation 12 au moyen d'un outil adapté, pour son implantation dans le matériau osseux de réception. L'empreinte de manœuvre 16 est disposée axialement au niveau de l'extrémité de l'organe de fixation 12.

Le pourtour de la tête de vis 13 comprend un filetage externe 17 destiné à coopérer avec le taraudage interne 8 des orifices diaphysaires 7a pour obtenir un verrouillage de la vis de fixation 12 sur la plaque support 1.

Une forme de réalisation possible d'un organe de fixation épiphysaire 20 est illustrée sur les figures 3 et 4.

Cet organe de fixation épiphysaire 20 se présente sous la forme d'un organe allongé canulé, c'est-à-dire muni d'un canal longitudinal axial (ce canal axial étant adapté au passage d'une broche de fixation, comme décrit plus loin dans la description).

Plus précisément, cet organe de fixation épiphysaire 20 comprend - une tête d'organe 21 dont une partie au moins du contour est destinée à venir en contact avec une partie du pourtour de l'un des orifices épiphysaire 7c, et - un corps d'organe 22, d'axe longitudinal 22'.

Un canal longitudinal axial 23 s'étend sur toute la longueur de l'organe de fixation épiphysaire 20 et débouche au niveau de ses deux extrémités, c'est-à-dire au niveau de la tête d'organe 21 et au niveau de l'extrémité libre du corps d'organe 22.

Une empreinte de tête 24 (ou empreinte de manœuvre 24), en creux, réalisée dans la tête d'organe 21, permet les manœuvres de rotation de l'organe de fixation 20 au moyen d'un outil adapté, pour son implantation dans le matériau osseux de réception. L'empreinte de manœuvre 24 est disposée axialement au niveau de l'extrémité de l'organe de fixation 20.

Avantageusement, l'empreinte de tête 16 des organes de fixation diaphysaires 12 et l'empreinte de tête 24 des organes de fixation épiphysaires 20 sont identiques pour que ces deux types d'organes de fixation 12, 20 puissent être manœuvrés en rotation par le même outil de manœuvre.

Le pourtour de la tête d'organe 21 comprend un filetage externe 25 destiné à coopérer avec le taraudage interne 10 des orifices épiphysaires 7c pour obtenir un verrouillage de l'organe de fixation 20 sur la plaque support 1.

Dans le mode de réalisation illustré, le corps d'organe 22 est muni (a) d'une partie proximale 26, située du côté de la tête d'organe 21, qui comporte un filetage externe 27 adapté pour venir se fixer par vissage dans le matériau osseux, et (b) d'une partie distale 28 dépourvue de filetage jusqu'à son extrémité libre. Ici, la longueur de la partie proximale filetée 26 est comprise entre 1/5 et 1/3 de la longueur du corps d'organe 22.

Dans une variante de réalisation, toute la longueur du corps d'organe 22 des organes de fixation 20 comporte un filetage externe.

Et selon encore une autre variante de réalisation, le corps d'organe 22 des organes de fixation 20 est lisse et ne comporte donc pas de filetage externe.

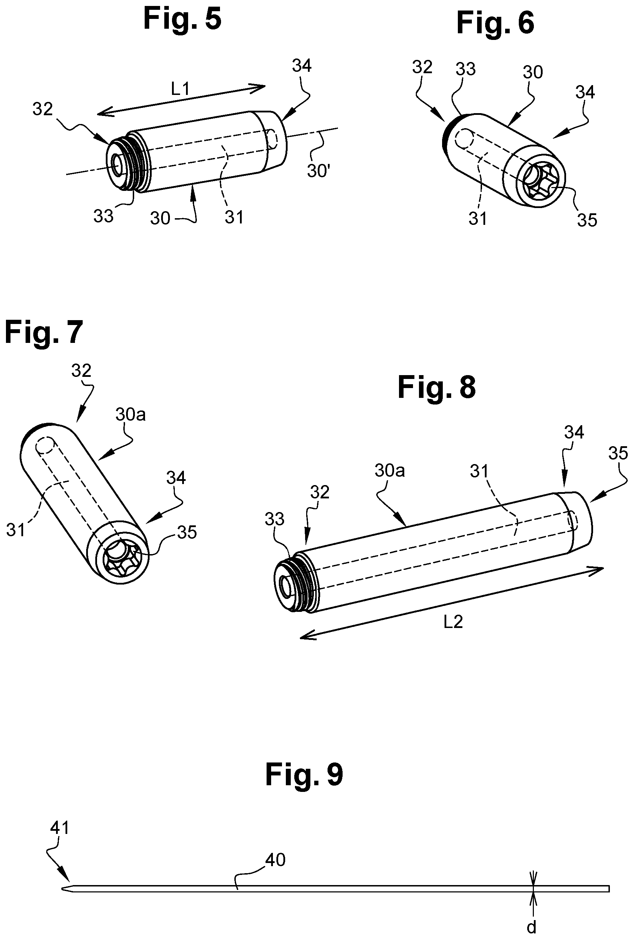

Une forme de réalisation possible d'un guide de visée 30 pour un ensemble chirurgical conforme à l'invention est illustrée sur les figures 5 et 6.

Ce guide de visée 30 se présente sous la forme générale d'un tube d'axe longitudinal 30', muni d'un orifice ou canal axial 31.

Une extrémité 32 de ce guide de visée 30 est munie d'un filetage externe 33 adapté à coopérer avec le taraudage 10 de l'un des orifices épiphysaires 7c. D'autre part, l'autre extrémité 34 (opposée à l'extrémité 32) du guide de visée 30 comprend une empreinte de manœuvre 35 pour permettre sa manœuvre en rotation autour de l'axe longitudinal 30' au moyen d'un outil de manœuvre adapté. L'empreinte de manœuvre 35 est disposée axialement au niveau de l'extrémité du guide de visée 30.

Cette empreinte de manœuvre 35 est identique à l'empreinte de tête 16 des organes de fixation diaphysaire 12 et à l'empreinte de tête 24 des organes de fixation épiphysaire 20 pour pouvoir être manœuvrée en rotation par le même outil de manœuvre.

Les figures 7 et 8 illustrent une variante de réalisation d'un guide de visée 30a pour un ensemble chirurgical conforme à l'invention. Ce guide de visée 30a est identique au guide de visée 30 décrit en relation avec les figures 5 et 6, excepté au niveau de sa longueur L2 qui est supérieure à la longueur L1 du guide de visée 30 décrit ci-dessus.

Une forme de réalisation possible d'une broche de fixation 40 pour un ensemble chirurgical conforme à l'invention est illustrée sur la figure 9.

Cette broche de fixation 40 est réalisée en métal, par exemple en inox ou en titane. Elle se présente sous la forme d'une tige lisse comportant une extrémité 41 en forme de pointe ; sa longueur peut être comprise entre 40 et 200mm et son diamètre d, constant, peut être compris entre 0,8 et 2,5mm.

Ce diamètre d de la broche de fixation 40 correspond, au jeu près, au diamètre du canal longitudinal axial 23 de l'organe de fixation épiphysaire 20 et au diamètre du canal axial 31 des guides de visée 30, 30a.

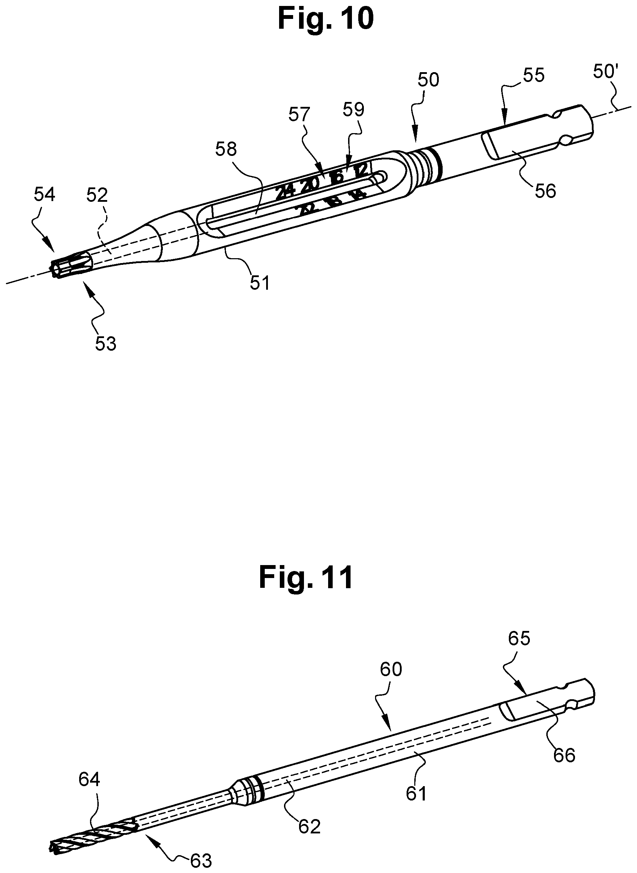

Une forme de réalisation possible d'un outil canulé de manœuvre 50 pour un ensemble chirurgical conforme à l'invention est illustrée sur la figure 10.

Cet outil de manœuvre 50 comprend un corps allongé 51, d'axe longitudinal 50', muni d'un canal axial 52. L'une de ses extrémités 53 comprend une tête de manœuvre 54 et son autre extrémité 55 comprend des moyens 56 adaptés pour sa fixation amovible sur un manche de manœuvre. La tête de manœuvre 54 est disposée axialement au niveau de l'extrémité 53 de l'outil de manœuvre 50.

La tête de manœuvre 54 est conçue pour pouvoir coopérer avec l'empreinte de manœuvre 35 des guides de visée 30, 30a, avec l'empreinte de tête 24 des organes de fixation épiphysaires 20 et avec l'empreinte de tête 16 des organes de fixation diaphysaires 12.

D'autre part, le diamètre du canal axial 52 correspond, au jeu près, au diamètre d des broches de fixation 40.

Entre ses deux extrémités 53 et 56, l'outil canulé de manœuvre 50 comporte des moyens de mesure 57 adaptés pour mesurer la profondeur d'enfoncement des broches de fixation 40 dans le matériau osseux de réception R, comme expliqué plus loin dans la suite de la description.

Ces moyens de mesure 57 comprennent une lumière 58 allongée autorisant un accès visuel à une partie du canal axial 52, associée à un système de graduation 59.

Ce système de graduation 59 est conçu, une fois l'outil de manœuvre 50 positionné sur une broche de fixation 40 enfoncée dans le matériau osseux R, avec sa tête de manœuvre 54 en prise dans l'empreinte de manœuvre d'un guide de visée 30, 30a associé, pour indiquer au praticien/chirurgien la profondeur d'enfoncement de la broche de fixation 40, par la position de l'extrémité libre de cette broche 40 en regard dudit système de graduation 59, ou au moyen d'un trait de marquage réalisé sur la longueur de la broche de fixation 40. Et la longueur d'enfoncement de la broche de fixation 40 indique au praticien/chirurgien la longueur de l'organe de fixation épiphysaire 20 à utiliser pour la fixation dans l'os.

On comprend par conséquent que cet outil canulé de manœuvre 50 remplit plusieurs fonctions : l'une de manœuvre en rotation des guides de visée et des organes de fixation, et une autre de mesure de profondeur d'enfoncement des broches de fixation.

Dans des variantes de réalisation, plusieurs outils de manœuvre peuvent être prévus, par exemple si les empreintes de manœuvre 16, 24 et 35 sont différentes ; ou encore si on souhaite disposer d'un outil de mesure de profondeur distinct de l'outil de manœuvre en rotation.

Une forme de réalisation possible d'un outil de forage canulé 60 pour un ensemble chirurgical conforme à l'invention est illustrée sur la figure 11.

Cet outil de forage canulé 60 consiste en un forêt destiné à coopérer avec un outillage d'entrainement en rotation, tel qu'un moteur chirurgical ou perceuse (non représenté(e)) pour constituer ensemble un matériel de forage.

L'outil de forage canulé 60 comprend un corps allongé 61 muni d'un orifice ou canal longitudinal axial 62. L'une de ses extrémités 63 comprend une tête de forage 64 et son autre extrémité 65 comprend des moyens 66 adaptés pour venir se fixer sur la tête de la perceuse (ou moteur chirurgical) associée, de manière à assurer l'entrainement en rotation de la tête de forage 64. Le diamètre du canal longitudinal axial 62 correspond, au jeu près, au diamètre d des broches de fixation 40.

L'ensemble chirurgical selon l'invention comprend encore avantageusement un outillage adapté pour enfoncer les broches de fixation 40 dans le matériau osseux R. Cet outillage peut consister en une perceuse (ou moteur chirurgical).

Et selon encore une autre particularité, cet ensemble chirurgical peut comprendre également un équipement d'arthroscopie adapté pour que le praticien/chirurgien puisse visualiser en direct notamment la réduction anatomique de la fracture, et aussi la mise en place des broches de fixation 40 et des organes de fixation épiphysaires 20 dans le matériau osseux R.

Cet équipement d'arthroscopie, non représenté et classique en lui-même, comprend une caméra miniaturisée à l'extrémité d'une fibre optique reliée à un écran.

Pour permettre la mise en œuvre d'une opération chirurgicale adaptée à la fracture en présence, l'ensemble chirurgical selon l'invention proposé au praticien comprend :
- une pluralité de plaques support d'ostéosynthèse 1 qui diffèrent par leur forme générale et/ou par leurs dimensions,
- une pluralité d'organes de fixation diaphysaires 12 et épiphysaires 20 de différentes formes et/ou dimensions,
- au moins un guide de visée 30, 30a, et de préférence plusieurs guides de visée 30, 30a (pour certains identiques entre eux et pour d'autres différents en longueur),
- plusieurs broches de fixation 40 de longueur identique ou non,
- au moins un outil 50 pour la mesure de la longueur des organes de fixation épiphysaires 20 à mettre en place, et pour la manoeuvre en rotation des différents organes de fixation 12, 20, ainsi que des guides de visée 30, 30a,
- un jeu d'outils de forage 60,
- un outillage de type perceuse ou moteur chirurgical, pour les outils de forage 60 et pour enfoncer les broches de fixation 40 dans le matériau osseux de réception R,
- un équipement d'arthroscopie pour visualiser la réduction anatomique de la fracture et aussi la mise en place notamment des broches de fixation 40, des organes de fixation épiphysaires 20 et des organes de fixation diaphysaires 12,
- éventuellement un jeu de guides de forage.

### Mode opératoire

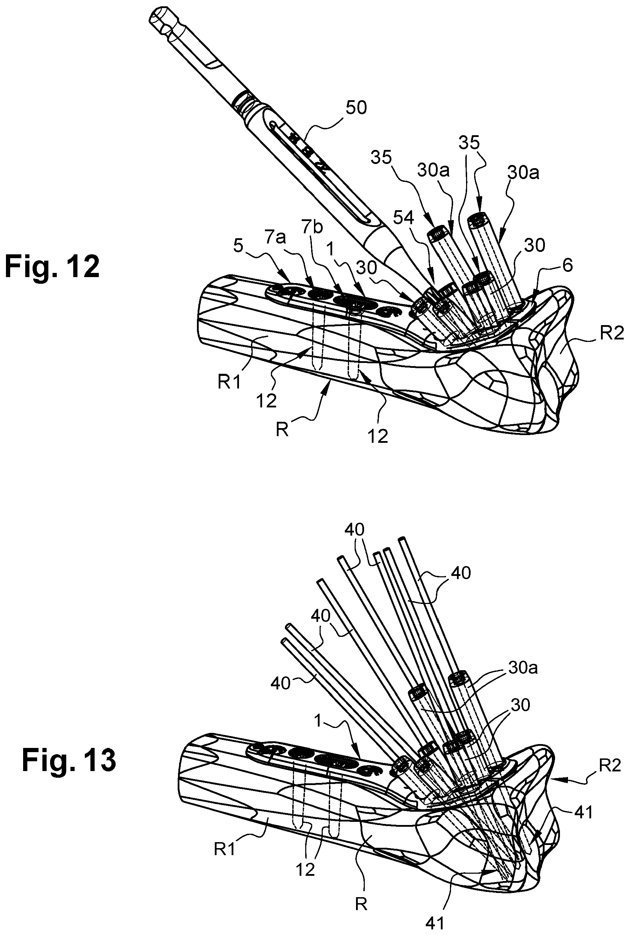

Les figures 12 à 17 illustrent en différentes étapes le mode opératoire pour la réduction d'une fracture osseuse au moyen d'un l'ensemble chirurgical conforme à l'invention décrit ci-dessus.

La figure 12 illustre un matériau osseux R (par exemple l'extrémité d'un radius) comprenant une partie diaphysaire R1 et une partie épiphysaire R2 ayant subi une fracture osseuse que l'on cherche à réduire.

Dans un premier temps, la partie diaphysaire 5 d'une plaque support d'ostéosynthèse 1 est fixée sur la partie diaphysaire R1 du matériau osseux R au moyen de vis de fixation diaphysaires 12, cela de sorte que la partie épiphysaire de plaque 6 soit située en regard de la fracture osseuse à réduire.

Dans l'exemple de réalisation représenté, une vis de fixation diaphysaire 12 est implantée dans le matériau osseux R via l'orifice diaphysaire 7b oblong, et une autre vis de fixation diaphysaire 12 est implantée dans le matériau osseux R via l'un des orifices diaphysaires 7a cylindrique. Tous les orifices diaphysaires 7a cylindriques peuvent être équipés d'une vis de fixation diaphysaire 12 pour optimiser la fixation de la plaque support d'ostéosynthèse 1.

Ces vis de fixation diaphysaires 12 sont mises en place au moyen d'un tournevis par coopération de sa tête de manœuvre avec l'empreinte de tête 16 desdites vis diaphysaires 12.

Une fois la partie épiphysaire de plaque 6 correctement positionnée en regard de la fracture osseuse à réduire, le praticien positionne les guides de visée 30, 30a sur cette partie épiphysaire de plaque 6. Pour cela, le filetage externe 33 de l'extrémité distale 32 des guides de visée 30, 30a est vissé sur le taraudage 10 des orifices épiphysaires 7c choisis. Ce vissage peut être réalisé par l'outil canulé de manœuvre 50 (dont la tête de manœuvre 54 coopère avec l'empreinte de manœuvre 35 des guides de visée 30, 30a).

Comme on peut le voir sur la figure 13, le praticien positionne ensuite les broches de fixation 40 en utilisant les guides de visée 30, 30a.

Chaque broche de fixation 40 passe dans le canal axial 31 de l'un des guides de visée 30, 30a et est guidée par ce dernier. L'enfoncement des broches de fixation 40 est réalisé par l'outillage adapté de type perceuse (ou moteur chirurgical) ; et cet enfoncement peut être réalisé sous arthroscopie de manière à ce que le praticien/chirurgien puisse visualiser de manière précise le positionnement de chaque broche de fixation 40.

L'enfoncement des broches de fixation 40 est réalisé jusqu'à ce que leur extrémité en forme de pointe 41 atteigne la corticale opposée de l'os. Cette opération de mise en place des broches de fixation 40 peut être réalisée sous arthroscopie.

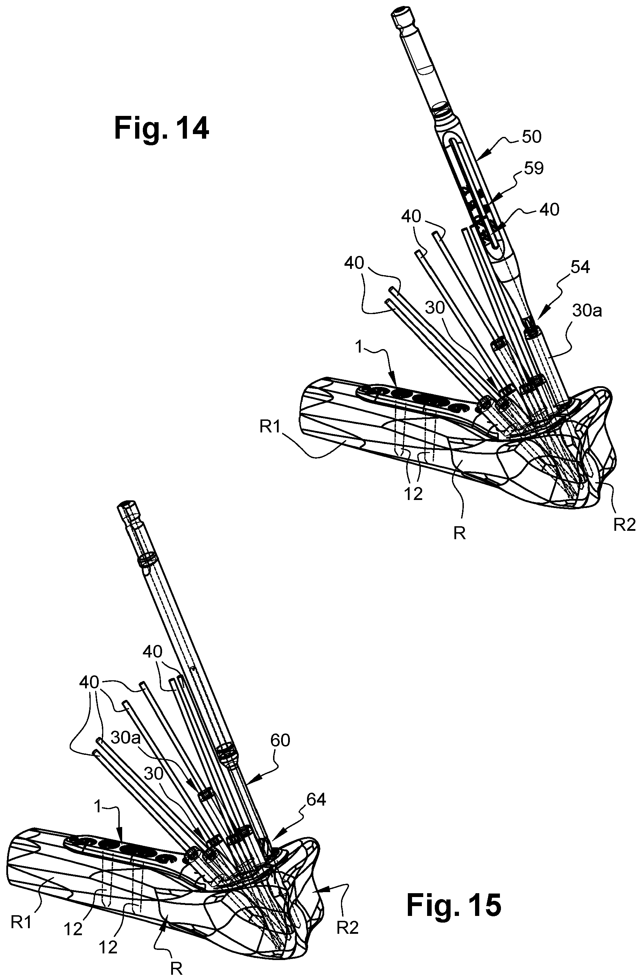

Ensuite, comme représenté sur la figure 14, le praticien positionne l'outil de manœuvre et de mesure canulé 50 sur une broche de fixation 40, avec son guide de visée 30, 30a associé toujours en place, pour mettre en œuvre sa fonction de « mesure de profondeur » afin de mesurer la profondeur d'enfoncement de cette broche de fixation, de manière à connaitre la longueur de l'organe de fixation épiphysaire 20 qui devra être utilisé ici.

Pour cela, comme mentionné ci-dessus, le praticien positionne l'outil de manœuvre 50 sur la broche de fixation 40 enfoncée dans le matériau osseux R, avec sa tête de manœuvre 54 en prise dans l'empreinte de manœuvre 35 du guide de visée 30, 30a ; et le système de graduation 59 associé à la lumière 58 allongée, en regard du positionnement de l'extrémité libre de la broche de fixation 40 (ou en regard d'un trait de marquage sur la longueur de cette broche) indique au praticien la profondeur d'enfoncement de la broche 40, et donc la longueur requise de l'organe de fixation épiphysaire 20 à utiliser par la suite.

Le praticien enlève ensuite le guide de visée 30, 30a au moyen de l'outil de manoeuvre canulé 50 (cet enlèvement est réalisé sans affecter le positionnement de la broche de fixation associée 40). Et il peut alors forer l'orifice de réception de l'organe de fixation épiphysaire 20 au moyen du matériel de forage comprenant l'outil de forage canulé 60, comme illustré sur la figure 15.

De préférence cette opération de forage est mise en œuvre sous arthroscopie ; elle est guidée par la broche de fixation 40 et elle est réalisée jusqu'à atteindre la corticale opposée (de préférence sans traverser cette corticale).

L'extrémité de la broche de fixation 40 reste de préférence enfoncée et maintenue par cette corticale.

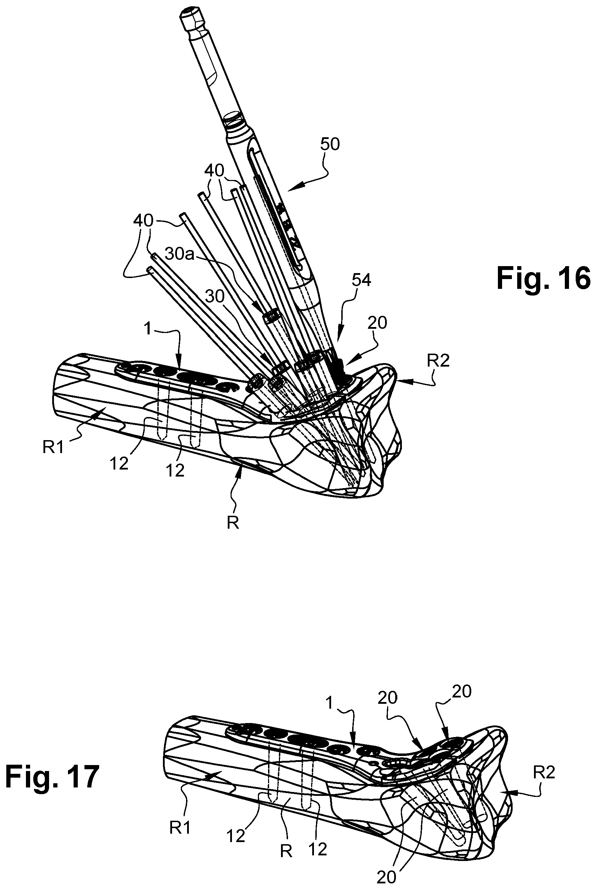

Une fois le forage réalisé, l'organe de fixation épiphysaire 20 choisi peut être implanté par enfoncement et/ou vissage dans le forage obtenu, comme on peut le voir sur la figure 16. Cette opération est réalisée par enfoncement et vissage au moyen de l'outil de manoeuvre canulé 50, l'organe de fixation épiphysaire 20 étant guidé par la broche de fixation 40.

L'organe de fixation épiphysaire 20 est verrouillé en position par vissage du filetage externe 25 de sa tête d'organe 21 sur le taraudage interne 10 de l'orifice épiphysaire 7c associé. La finalisation de ce vissage peut être réalisée, après retrait de la broche de fixation 40, au moyen d'un tournevis plein (non canulé).

Cette opération de mise en place de l'organe de fixation épiphysaire 20 peut être réalisée sous arthroscopie.

Toutes les broches nécessaires à la réduction de la fracture peuvent être mises en place. Ensuite, les opérations précitées de prise de mesure de profondeur (figure 14), d'enlèvement du guide de visée 30, 30a, de forage (figure 15) et de mise en place de l'organe de fixation épiphysaire 20 (figure 16), sont réalisées pour chacune des broches de fixation 40, pour obtenir la plaque support d'ostéosynthèse 1 équipée de tous les organes de fixation épiphysaires 20 nécessaires à la réduction de la fracture osseuse en présence, telle qu'illustrée sur la figure 17.

A titre de variante de réalisation, un seul guide de visée 30, 30a peut être proposé dans l'ensemble chirurgical selon l'invention, cet unique guide de visée étant réutilisé après le positionnement de chaque broche de fixation 40 et la mise en place d'un organe de fixation épiphysaire 20.

De préférence malgré tout, on utilise plusieurs guides de visée 30, 30a, dont le ou les plus longs peuvent être utilisés pour écarter certains tissus, nerfs ou autres, lors de leur positionnement sur la plaque d'ostéosynthèse, de manière à faciliter l'accès à la zone osseuse à traiter.

D'une manière générale, un tel ensemble chirurgical permet de faciliter et d'optimiser la stabilisation temporaire d'une fracture articulaire.

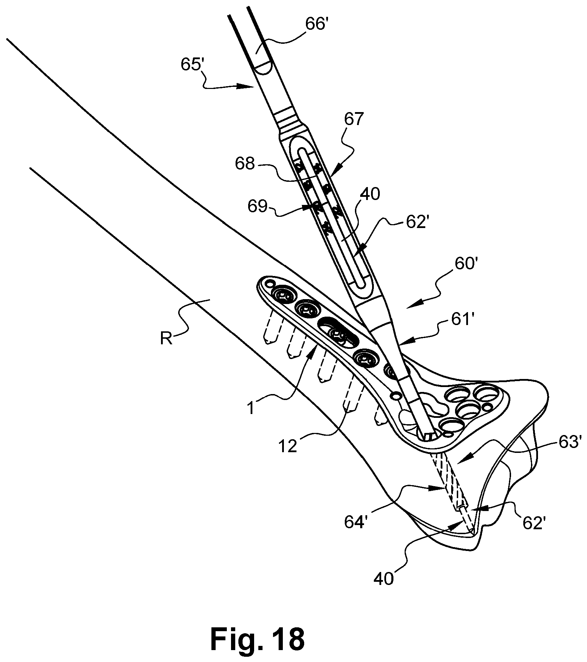

Une variante de réalisation possible d'un outil de forage canulé 60' pour un ensemble chirurgical conforme à l'invention est illustrée sur la figure 18.

Cet outil de forage canulé 60' consiste en un forêt canulé permettant le forage du matériau osseux sur une broche de fixation 40 en place, et qui est équipé de moyens de mesure adaptés pour renseigner le praticien/chirurgien sur la profondeur de forage par rapport à l'extrémité de la broche.

L'outil de forage canulé 60' comprend un corps allongé 61' muni d'un orifice ou canal longitudinal axial 62'. L'une de ses extrémités 63' comprend une tête de forage 64' et son autre extrémité 65' comprend des moyens 66' adaptés pour venir se fixer sur la tête d'une perceuse (ou moteur chirurgical) associée, de manière à assurer l'entrainement en rotation de la tête de forage 64'.

Le diamètre du canal longitudinal axial 62' correspond, au jeu près, au diamètre des broches de fixation 40.

Entre ses deux extrémités 63' et 65', l'outil de forage canulé 60' comporte les moyens de mesure précités, repérés 67, adaptés pour renseigner le praticien sur la profondeur du forage par rapport à l'extrémité de la broche 40 sur laquelle le forage est réalisé.

Ces moyens de mesure 67 comprennent une lumière 68 allongée autorisant un accès visuel à une partie du canal axial 62', associée à un système de graduation 69.

Ce système de graduation 69 est conçu pour indiquer au praticien/chirurgien où en est la profondeur de forage par rapport à l'extrémité de la broche 40 enfoncée dans le matériau osseux, cela par la position de l'extrémité libre de cette broche 40 en regard dudit système de graduation 69, ou au moyen d'un trait de marquage réalisé sur la longueur de la broche de fixation 40.

Par exemple, le praticien/chirurgien peut choisir de réaliser le forage jusqu'à la pointe de la broche 40, ou juste un peu avant d'arriver au niveau de cette pointe de broche.

On comprend par conséquent qu'un tel outil de forage canulé 60' permet d'assurer un forage en vue de la mise en place des organes de fixation, et permet aussi de déterminer de la profondeur de forage par rapport à l'extrémité enfoncée des broches de fixation 40.

## Revendications

1. Ensemble chirurgical adapté pour la réduction d'une fracture osseuse, en particulier d'une fracture située au niveau des épiphyses osseuses, comprenant :
- une plaque support d'ostéosynthèse (1) comprenant une face inférieure (2) et une face supérieure (3), ladite face inférieure (2) étant destinée à être positionnée contre le matériau osseux (R) de réception, laquelle plaque support (1) comprend une partie de corps allongée (5), dite partie diaphysaire de plaque (5), prolongée par une partie de tête monobloc (6), dite partie épiphysaire de plaque (6), ladite partie diaphysaire de plaque (5) comprenant une pluralité d'orifices traversants (7a, 7b), dits orifices diaphysaires (7a, 7b), et ladite partie épiphysaire de plaque (6) comprenant une pluralité d'orifices traversants (7c), dits orifices épiphysaires (7c), lesquels orifices diaphysaires (7a, 7b) et orifices épiphysaires (7c) sont aptes à autoriser chacun l'accueil d'un organe de fixation allongé (12, 20), certains au moins desdits orifices épiphysaires (7c) comprenant un taraudage interne (10),
- un jeu d'organes de fixation (12) dans le matériau osseux (R) destinés à être insérés dans lesdits orifices diaphysaires (7a, 7b), dits organes de fixation diaphysaires (12), pour fixer ladite partie diaphysaire de plaque (5) à la surface du matériau osseux (R),
- un jeu d'organes de fixation (20) dans le matériau osseux (R) destinés à être insérés dans lesdits orifices épiphysaire (7c), dits organes de fixation épiphysaires (20), adaptés en particulier pour permettre la réduction recherchée de la fracture osseuse,
- des moyens (50) pour la manœuvre en rotation desdits organes de fixation diaphysaires (7a, 7b) et desdits organes de fixation épiphysaires (7c), de sorte à assurer leur ancrage par vissage dans le matériau osseux (R),
- au moins un guide de visée (30, 30a) de forme tubulaire, muni d'un canal axial (31) s'étendant le long de son axe longitudinal (30'), dont une extrémité (32) est munie d'un filetage externe (33) adapté à coopérer avec le taraudage (10) de l'un desdits orifices épiphysaires (7c), et dont l'autre extrémité (34) comprend une empreinte de manœuvre (35),
- un outil (50) de manœuvre en rotation, conçu pour coopérer avec l'empreinte de manœuvre (35) dudit au moins un guide de visée (30, 30a), pour le vissage de son extrémité filetée (32, 33) dans l'un desdits orifices épiphysaires (7c), et son dévissage, lequel outil (50) de manœuvre en rotation est muni d'un canal axial (52) s'étendant le long de son axe longitudinal (50'),
- une pluralité de broches de fixation (40), conçues pour coopérer avec ledit au moins un guide de visée (30, 30a), c'est-à-dire chacune apte à être insérée dans le canal axial (31) dudit au moins un guide de visée (30, 30a) pour assurer son guidage longitudinal et chacune apte à assurer la réduction recherchée temporaire au moins partielle de la fracture osseuse,
- un matériel de forage comprenant un outil de forage canulé (60, 60') muni d'un canal longitudinal axial (62, 62'), adapté pour pouvoir être inséré sur l'une desdites broches de fixation (40) positionnée au travers de l'un desdits orifices épiphysaires (7c) et pour pouvoir forer le matériau osseux (R),
lesdits organes de fixation épiphysaires (20) se présentant au moins pour certains sous la forme d'organes de fixation canulés (20), munis d'un canal longitudinal axial (22) adapté au passage de l'une desdites broches de fixation (40),
lesdits moyens (50) pour la manœuvre en rotation desdits organes de fixation épiphysaires (20) se présentant sous la forme d'un outil canulé de manœuvre (50) muni d'un canal longitudinal axial (52) adapté au passage de l'une desdites broches de fixation (40), et dont l'une des extrémités (53, 54) est structurée pour coopérer avec une empreinte de tête (24) de forme complémentaire ménagée dans une tête d'organe (21) desdits organes de fixation épiphysaires (20),
**caractérisé en ce qu'**il comprend en outre :
des moyens de mesure (50, 57, 58, 59), conçus pour mesurer la profondeur d'enfoncement desdites broches de fixation (40) dans le matériau osseux de réception (R), lesquels moyens de mesure (50, 57, 58, 59) sont ménagés sur ledit outil canulé (50) de manœuvre en rotation des guides de visée (30, 30a) et/ou des organes de fixation épiphysaires (20).

2. Ensemble chirurgical selon la revendication 1, **caractérisé en ce que** lesdits organes de fixation diaphysaires (12) et lesdits organes de fixation épiphysaires (20) comprennent une même empreinte de tête (16, 24) de sorte à pouvoir être manœuvrés en rotation par le même outil canulé de manœuvre (50).

3. Ensemble chirurgical selon l'une quelconque des revendications 1 ou 2, **caractérisé en ce que** ledit au moins un guide de visée (30, 30a) comprend une empreinte de manœuvre (35) identique à l'empreinte de tête (24) desdits organes de fixation épiphysaires (20) de sorte à pouvoir être manœuvré en rotation par le même outil canulé de manœuvre (50).

4. Ensemble chirurgical selon l'une quelconque des revendications 1 à 3, **caractérisé en ce que** l'outil de forage canulé (60') comprend des moyens de mesure (67, 68, 69) pour déterminer la profondeur du forage par rapport à l'extrémité de la broche de fixation (40) sur laquelle le forage est réalisé.

5. Ensemble chirurgical selon l'une quelconque des revendications 1 à 4, **caractérisé en ce qu'**il comprend un équipement d'arthroscopie adapté pour permettre la visualisation notamment de la réduction anatomique recherchée, et de la mise en place des broches de fixation (40) et des organes de fixation épiphysaires (20) dans le matériau osseux (R).

6. Ensemble chirurgical selon l'une quelconque des revendications 1 à 5, **caractérisé en ce que** lesdits organes de fixation épiphysaires (20) comprennent - une tête d'organe (21) munie d'un filetage externe (25), lequel filetage externe (25) est adapté pour coopérer avec le taraudage (10) des orifices épiphysaires (20), et - un corps d'organe (22), prolongeant ladite tête d'organe (21), muni (a) d'une partie proximale (26) comportant un filetage externe (27) adapté pour venir se fixer par vissage dans le matériau osseux (R), et (b) d'une partie distale (28) dépourvue de filetage.

7. Ensemble chirurgical selon l'une quelconque des revendications 1 à 5, **caractérisé en ce que** lesdits organes de fixation épiphysaires (20) comprennent - une tête d'organe (21) munie d'un filetage externe (25), lequel filetage externe (25) est adapté pour coopérer avec le taraudage (10) des orifices épiphysaire (7c), et - un corps d'organe, prolongeant ladite tête d'organe, lequel corps d'organe est muni d'un filetage sur toute sa longueur.

8. Ensemble chirurgical selon l'une quelconque des revendications 1 à 5, **caractérisé en ce que** lesdits organes de fixation épiphysaires (20) comprennent - une tête d'organe (21) munie d'un filetage externe (25), lequel filetage externe (25) est adapté pour coopérer avec le taraudage (10) des orifices épiphysaire (7c), et - un corps d'organe prolongeant ladite tête d'organe, lequel corps d'organe est lisse, dépourvu de filetage.

9. Ensemble chirurgical selon l'une quelconque des revendications 1 à 8, **caractérisé en ce qu'**il comprend un outillage de type perceuse ou moteur chirurgical adapté pour enfoncer lesdites broches de fixation (40) dans le matériau osseux (R).

10. Ensemble chirurgical selon l'une quelconque des revendications 1 à 9, **caractérisé en ce qu'**il comprend un premier type de guide de visée (30) d'une longueur (L1), et au moins un deuxième type de guide de visée (30a) d'une longueur (L2) différente de (L1).

## Patentansprüche

1. Chirurgische Anordnung zur Reduzierung einer Knochenfraktur, insbesondere einer Fraktur in Höhe der Knochenepiphysen, mit:
- einer Osteosynthese-Stützplatte (1) mit einer Unterseite (2) und einer Oberseite (3), wobei die Unterseite (2) dazu bestimmt ist, am Empfängerknochenmaterial (R) angeordnet zu sein, wobei die Stützplatte (1) einen als diaphysären Plattenteil (5) bezeichneten länglichen Körperteil (5) aufweist, der durch einen als epiphysären Plattenteil (6) bezeichneten Kopfteil (6) in einem Stück verlängert ist, wobei der diaphysäre Plattenteil (5) mehrere als diaphysäre Löcher (7a, 7b) bezeichnete Durchgangslöcher (7a, 7b) aufweist, und wobei der epiphysäre Plattenteil (6) mehrere als epiphysäre Löcher (7c) bezeichnete Durchgangslöcher (7c) aufweist, wobei die diaphysären Löcher (7a, 7b) und die epiphysären Löcher (7c) geeignet sind, jeweils die Aufnahme eines länglichen Befestigungsorgans (12, 20) zu ermöglichen, wobei mindestens einige der epiphysären Löcher (7c) ein Innengewinde (10) aufweisen,
- einem Satz als diaphysäre Befestigungsorgane (12) bezeichnete Organe (12) für die Befestigung im Knochenmaterial (R), die dazu bestimmt sind, in die diaphysären Löcher (7a, 7b) eingesetzt zu werden, um den diaphysären Plattenteil (5) auf der Oberfläche des Knochenmaterials (R) zu befestigen,
- einem Satz als epiphysäre Befestigungsorgane (20) bezeichnete Organe (20) für die Befestigung im Knochenmaterial (R), die dazu bestimmt sind, in die epiphysären Löcher (7c) eingesetzt zu werden, um die gewollte Reduzierung der Knochenfraktur zu ermöglichen,
- Mitteln (50) zur drehenden Betätigung der diaphysären Befestigungsorgane (7a, 7b) und der epiphysären Befestigungsorgane (7c), um deren Verankerung im Knochenmaterial (R) durch Verschrauben sicherzustellen,
- mindestens einer rohrförmigen Zielhilfe (30, 30a), die mit einem sich entlang ihrer Längsachse (30') erstreckenden axialen Kanal (31) versehen ist, deren eines Ende (32) mit einem Außengewinde (33) versehen ist, das geeignet ist, mit dem Innengewinde (10) eines der epiphysären Löcher (7c) zusammenzuwirken, und deren anderes Ende (34) eine Betätigungsvertiefung (35) aufweist,
- einem Werkzeug (50) zur drehenden Betätigung, das dazu ausgelegt ist, mit der Betätigungsvertiefung (35) der mindestens einen Zielhilfe (30, 30a) zum Einschrauben ihres mit einem Gewinde versehenen Endes (32, 33) in eins der epiphysären Löcher (7c) und zum Herausschrauben zusammenzuwirken, wobei das Werkzeug (50) zur drehenden Betätigung mit einem sich entlang seiner Längsachse (50') erstreckenden axialen Kanal (52) versehen ist,
- einer Anzahl Befestigungsstifte (40), die dazu ausgelegt sind, mit der mindestens einen Zielhilfe (30, 30a) zusammenzuwirken, das heißt, die jeweils geeignet sind, in den axialen Kanal (31) der mindestens einen Zielhilfe (30, 30a) eingesetzt zu werden, um deren Längsführung sicherzustellen, und jeweils geeignet sind, die gewollte vorübergehende mindestens teilweise Reduzierung der Knochenfraktur zu erzielen,
- einer Bohrausrüstung mit einem mit einem axialen Längskanal (62, 62') versehenen Bohrwerkzeug (60, 60'), das dazu ausgelegt ist, auf einen der durch eins der epiphysären Löcher (7c) geführten Befestigungsstifte (40) aufgesetzt zu werden, und um in das Knochenmaterial(R) bohren zu können,
wobei mindestens einige der epiphysären Befestigungsorgane (20) in Form von kanülierten Befestigungsorganen (20) vorliegen, die mit einem axialen Längskanal (22) versehen sind, der für das Hindurchführen eines der Befestigungsstifte (40) geeignet ist,
wobei die Mittel (50) für die drehende Betätigung der epiphysären Befestigungsorgane (20) in Form eines mit einem axialen längsgerichteten Kanal versehenen kanülierten Betätigungswerkzeugs (50) vorliegen, das für das Hindurchführen eines der Befestigungsstifte (40) geeignet ist und von dem das eine der Enden (53, 54) so strukturiert ist, daß es mit einer in einem Organkopf (21) der besagten epiphysären Befestigungsorgane (20) angeordneten Kopfvertiefung (24) komplementärer Form zusammenwirkt,
**dadurch gekennzeichnet, daß** sie außerdem
Meßmittel (50, 57, 58, 59) aufweist, die dazu ausgelegt sind, die Eindringtiefe der Befestigungsstifte (40) in das Empfangsknochenmaterial (R) zu messen, wobei die Meßmittel (50, 57, 58, 59) auf dem kanülierten Werkzeug (50) für die drehende Betätigung der Zielhilfen (30, 30a) und/oder der epiphysären Befestigungsorgane (20) eingerichtet sind.

2. Chirurgische Anordnung gemäß Anspruch 1, **dadurch gekennzeichnet, daß** die diaphysären Befestigungsorgane (12) und die epiphysären Befestigungsorgane (20) eine gleiche Kopfvertiefung (24) aufweisen, um mit dem gleichen kanülierten Betätigungswerkzeug (50) betätigt zu werden.

3. Chirurgische Anordnung gemäß einem der Ansprüche 1 oder 2, **dadurch gekennzeichnet, daß** die mindestens eine Zielhilfe (30, 30a) eine Betätigungsvertiefung (35) aufweist, die mit der Kopfvertiefung (24) der epiphysären Betätigungsorgane (20) identisch ist, um mit dem gleichen kanülierten Betätigungswerkzeug (50) betätigt zu werden.

4. Chirurgische Anordnung gemäß einem der Ansprüche 1 bis 3, **dadurch gekennzeichnet, daß** das kanülierte Bohrwerkzeug (60') Meßmittel (67, 68, 69) zum Bestimmen der Bohrtiefe im Bezug auf das Ende des Befestigungsstifts (40), auf dem die Bohrung erfolgt, aufweist.

5. Chirurgische Anordnung gemäß einem der Ansprüche 1 bis 4, **dadurch gekennzeichnet, daß** sie eine arthroskopische Ausrüstung aufweist, die geeignet ist, insbesondere die gewollte anatomische Reduktion und die Anordnung der Befestigunsstifte (40) und der epiphysären Befestigungsorgane (20) im Knochenmaterial (R) betrachtbar zu machen.

6. Chirurgische Anordnung gemäß einem der Ansprüche 1 bis 5, **dadurch gekennzeichnet, daß** die epiphysären Befestigungsorgane (20) - einen mit einem Außengewinde (25) versehenen Organkopf (21), wobei das Außengewinde (25) geeignet ist, mit dem Innengewinde (10) der epiphysären Löcher (20) zusammenzuwirken, und - einen Organkörper (22), der den Organkopf (21) verlängert und mit (a) einem proximalen Teil (26) mit einem Außengewinde (27), das geeignet ist, durch Einschrauben im Knochenmaterial (R) befestigt zu werden, und (b) einen distalen Teil (28) ohne Gewinde versehen ist, aufweisen.

7. Chirurgische Anordnung gemäß einem der Ansprüche 1 bis 5, **dadurch gekennzeichnet, daß** die epiphysären Befestigungsorgane (20) - einen mit einem Außengewinde (25) versehenen Organkopf (21), wobei das Außengewinde (25) geeignet ist, mit dem Innengewinde (10) der epiphysären Löcher (7c) zusammenzuwirken, und - einen Organkörper, der den Organkopf verlängert, wobei der Organkopf über seine gesamte Länge mit einem Gewinde versehen ist, aufweisen.

8. Chirurgische Anordnung gemäß einem der Ansprüche 1 bis 5, **dadurch gekennzeichnet, daß** die epiphysären Befestigungsorgane (20) - einen mit einem Außengewinde (25) versehenen Organkopf (21), wobei das Außengewinde (25) geeignet ist, mit dem Innengewinde (10) der epiphysären Löcher (7c) zusammenzuwirken, und - einen Organkörper, der den Organkopf verlängert, wobei der Organkörper glatt und ohne Gewinde ist, aufweisen.

9. Chirurgische Anordnung gemäß einem der Ansprüche 1 bis 8, **dadurch gekennzeichnet, daß** sie ein Werkzeug vom Typ einer Bohrmaschine oder eines chirurgischen Motors aufweist, das geeignet ist, die Befestigungsstifte (40) im Knochenmaterial (R) zu versenken.

10. Chirurgische Anordnung gemäß einem der Ansprüche 1 bis 9, **dadurch gekennzeichnet, daß** sie einen ersten Zielhilfetyp (30) einer Länge (L1) und mindestens einen zweiten Typ Zielhilfe (30a) einer von der Länge (L1) verschiedenen Länge (L2) aufweist.

## Claims

1. A surgical assembly for reducing a bone fracture, in particular a fracture located at the bone epiphyses, comprising:
- an osteosynthesis support plate (1) comprising a lower face (2) and an upper face (3), said lower face (2) being intended to be positioned against the receiving bone material (R), said support plate (1) comprising an elongated body portion (5), called the diaphyseal plate portion (5), extended by an integral head portion (6), called the epiphyseal plate portion (6), said diaphyseal plate portion (5) comprising a plurality of through-holes (7a, 7b), called diaphyseal holes (7a, 7b), and said epiphyseal plate portion (6) comprising a plurality of through-holes (7c), called epiphyseal holes (7c), said diaphyseal holes (7a, 7b) and epiphyseal holes (7c) each adapted for accommodating an elongated fixing member (12, 20), certain at least of said epiphyseal holes (7c) comprising an internal thread (10),
- a set of members (12) for the fixing into the bone material (R) intended to be inserted into said diaphyseal holes (7a, 7b), called the diaphyseal fixing members (12), to fix said diaphyseal plate portion (5) to the surface of the bone material (R),
- a set of members (20) for the fixing into the bone material (R), intended to be inserted into said epiphyseal holes (7c), called the epiphyseal fixing members (20), adapted in particular to allow the desired reduction of the bone fracture,
- means (50) for rotating said diaphyseal fixing members (7a, 7b) and said epiphyseal fixing members (7c), in such a way as to ensure their anchoring by screwing into the bone material (R),
- at least one sighting guide (30, 30a) of tubular shape, provided with an axial channel (31) extending along its longitudinal axis (30'), an end (32) of which being provided with an external thread (33) adapted to cooperate with the internal thread (10) of one of said epiphyseal holes (7c), and the other end (34) of which has an operating recess (35),
- a rotation operating tool (50), designed to cooperate with the operating recess (35) of said at least one sighting guide (30, 30a), for the screwing of its threaded end (32, 33) into one of said epiphyseal holes (7c), and the unscrewing thereof, said rotation operating tool (50) being provided with an axial channel (52) extending along its longitudinal axis (50'),
- a plurality of fixing pins (40), designed to cooperate with said at least one sighting guide (30, 30a), that is to say each adapted to be inserted into the axial channel (31) of said at least one sighting guide (30, 30a) to ensure the longitudinal guiding thereof and each adapted to ensure the desired, at least partial, temporary reduction of the bone fracture,
- drilling equipment comprising a cannulated drilling tool (60, 60') provided with an axial longitudinal channel (62, 62'), suitable for being inserted on one of said fixing pins (40) positioned through one of said epiphyseal holes (7c) and for drilling the bone material (R),
said epiphyseal fixing members (20) being, at least for some of them, in the form of cannulated fixing members (20), provided with an axial longitudinal channel (22) suitable for the passage of one of said fixing pins (40),
said means (50) for rotating said epiphyseal fixing members (20) being in the form of a cannulated operating tool (50) provided with an axial longitudinal channel (52) suitable for the passage of one of said fixing pins (40), and one end (53, 54) of which is structured to cooperate with a head recess (24) of complementary shape formed in a member head (21) of said epiphyseal fixing members (20),
**characterized in that** it comprises also:
measurement means (50, 57, 58, 59), designed to measure the driving depth of said fixing pins (40) into the receiving bone material (R),
wherein said measurement means (50, 57, 58, 59) are arranged on said cannulated operating tool (50) of the sighting guide (30, 30a) and/or epiphyseal fixing members (20).

2. The surgical assembly according to claim 1, **characterized in that** said diaphyseal fixing members (12) and said epiphyseal fixing members (20) have a same head recess (16, 24) in such a way that they can be rotated by the same cannulated operating tool (50).

3. The surgical assembly according to any one of claims 1 or 2, **characterized in that** said at least one sighting guide (30, 30a) has an operating recess (35) identical to the head recess (24) of said epiphyseal fixing members (20) in such a way that they can be rotated by the same cannulated operating tool (50).

4. The surgical assembly according to any one of claims 1 to 3, **characterized in that** the cannulated drilling tool (60') comprises measurement means (67, 68, 69) for determining the drilling depth with respect to the end of the fixing pin (40) on which the drilling is made.

5. The surgical assembly according to any one of claims 1 to 4, **characterized in that** it comprises arthroscopy equipment adapted to allow viewing in particular the desired anatomical reduction and the positioning of the fixing pins (40) and the epiphyseal fixing members (20) into the bone material (R).

6. The surgical assembly according to any one of claims 1 to 5, **characterized in that** said epiphyseal fixing members (20) comprise - a member head (21) provided with an external thread (25), said external thread (25) being adapted to cooperate with the internal thread (10) of the epiphyseal holes (7c), and - a member body (22), extending said member head (21), provided with (a) a proximal portion (26) having an external thread (27) suitable for being fixed by screwing into the bone material (R), and (b) a distal portion (28) with no thread.

7. The surgical assembly according to any one of claims 1 to 5, **characterized in that** said epiphyseal fixing members (20) comprise - a member head (21) provided with an external thread (25), said external thread (25) being adapted to cooperate with the internal thread (10) of the epiphyseal holes (7c), and - a member body, extending said member head, said member body being provided with a thread over its whole length.

8. The surgical assembly according to any one of claims 1 to 5, **characterized in that** said epiphyseal fixing members (20) comprise - a member head (21) provided with an external thread (25), said external thread (25) being adapted to cooperate with the internal thread (10) of the epiphyseal holes (7c), and - a member body, extending said member head, said member body being smooth, devoid of thread.

9. The surgical assembly according to any one of claims 1 to 8, **characterized in that** it comprises a tool of the drill or surgical motor type, suitable for driving said fixing pins (40) into the bone material (R).

10. The surgical assembly according to any one of claims 1 to 9, **characterized in that** it comprises a first type of sighting guide (30) of length (L1), and at least one second type of sighting guide (30a) of length (L2) different from (L1).
